# EUROPÄISCHE PATENTSCHRIFT

(11) **EP 1 776 034 B1**
(45) Veröffentlichungstag und Bekanntmachung des Hinweises auf die Patenterteilung: **05.09.2018**
(21) Anmeldenummer: 05766830.3
(22) Anmeldetag: 18.07.2005
(51) Int. Cl.: A61B 3/13, A61B 90/30

(54) **BELEUCHTUNGSEINRICHTUNG SOWIE BEOBACHTUNGSEINRICHTUNG**
ILLUMINATION DEVICE AND OBSERVATION DEVICE
DISPOSITIF D'ECLAIRAGE ET DISPOSITIF D'OBSERVATION

(30) Priorität: 06.08.2004 DE 102004038372; 18.10.2004 DE 102004050651
(43) Veröffentlichungstag der Anmeldung: 25.04.2007
(60) Teilanmeldung: 11183927.0
(73) Patentinhaber: Carl Zeiss Meditec AG, 07745 Jena (DE)
(72) Erfinder: REIMER, Peter, 73479 Ellwangen (DE); ABRAMOWSKY, Heinz, 89537 Geigen (Brenz) (DE); KOLSTER, Daniel, 73447 Oberkochen (DE); STRÄHLE, Fritz, 73540 Heubach (DE); ABELE, Alfons, 73527 Schwäbisch-Gmünd (DE)
(74) Vertreter: Müller, Thomas
(86) Internationale Anmeldenummer: PCT/EP2005/007803
(87) Internationale Veröffentlichungsnummer: WO 2006/015690

(56) Entgegenhaltungen:
- DE-A1- 4 326 761
- DE-A1- 4 417 273

## Beschreibung

Die vorliegende Erfindung betrifft ein Operationsmikroskop für die Ophthalmologie.

Ein ophthalmologisches Operationsmikroskop wird beispielsweise für eine spezielle Anwendung in der Augenchirurgie eingesetzt, nämlich der Kataraktchirurgie.

Bei der Kataraktchirurgie wird eine - beispielsweise durch den grauen Star getrübte - Augenlinse durch eine Kunstlinse ersetzt.

Die Augenlinse eines Auges befindet sich in einer dünnen Umhüllung, der so genannten Linsenkapsel. Zur Entfernung der Augenlinse wird durch einen dünnen Schnitt in die Linsenkapsel ein Zugang zur Augenlinse geschaffen und die Augenlinse mit einem mikrochirurgischen Gerät zunächst in kleine Einzelstücke zerteilt, die dann mittels einer Absaugvorrichtung entfernt werden.

Dieser Vorgang findet unter mikroskopischer Beobachtung - beispielsweise unter stereomikroskopischer Beobachtung - unter Einsatz einer für solche Eingriffe speziell ausgelegten Beleuchtungseinrichtung statt. Diese Beleuchtungseinrichtung stellt sowohl eine für die Ausleuchtung des gesamten Operationsfelds notwendige Umfeldbeleuchtung als auch eine für die Kataraktoperation entscheidend wichtige rote Hintergrundbeleuchtung für das eigentliche auf den Pupillenbereich der Augenlinse begrenzte Operationsfeld dar. Diese rote Hintergrundbeleuchtung rührt von dem Anteil des Beleuchtungslichts her, der über die transparenten Augenmedien schließlich auf die wegen einer guten Durchblutung rot erscheinenden Netzhaut trifft, von dieser zurückgestreut wird und dann natürlich über das Operationsmikroskop auch vom Chirurgen als rot erscheinende Hintergrundbeleuchtung beobachtet werden kann. Diese in der Kataraktchirurgie ganz charakteristische rote Hintergrundbeleuchtung ist in Fachkreisen allgemein unter dem Begriff "roter Reflex" bekannt.

Für eine optimale Erkennung der für die Kataraktoperation relevanten Details erweist sich für den Operateur eine möglichst homogene rote Hintergrundbeleuchtung als eine notwendige Voraussetzung. Eine erste Anforderung an die Beleuchtungseinrichtung ist also eine möglichst gute Homogenität des roten Reflexes über die gesamte Patientenpupille zu gewährleisten.

Zur vollständigen Beseitigung der Linsenreste der in winzige Teilstücke zerkleinerten Augenlinse und zur guten Erkennung von durchsichtigen Membranen, beispielsweise von der Linsenkapsel, muss eine weitere Anforderung erfüllt werden, nämlich eine gute Kontrastierung von Phasenobjekten und zwar möglichst auch über die gesamte Patientenpupille.

In der Vergangenheit sind im Zusammenhang mit der Erzeugung einer solchen roten Hintergrundbeleuchtung bereits verschiedene Lösungen bekannt geworden.

In der US-A-4,779,968 ist eine koaxiale Beleuchtung für ein Operationsmikroskop beschrieben. Gemäß dieser Lösung ist ein Beleuchtungsmodul vorgesehen, das als Zusatzbaustein an vorhandene Operationsmikroskope nachträglich eingebaut werden kann. Dieser Zusatzbaustein wird vorzugsweise objektseitig unterhalb des Hauptobjektivs der Beobachtungseinrichtung angebracht. Die Beleuchtungseinkopplung auf der Mikroskopachse erfolgt entweder mit einer Teilerplatte oder einem Teilerwürfel.

In der DE 40 28 605 C2 ist eine Beleuchtungseinrichtung für ein Operationsmikroskop beschrieben, welches eine Kombination von Null-Grad-, Koaxial- und Schrägbeleuchtung zulässt. Dazu verfügt die Beleuchtungseinrichtung über verschiebbare Teilspiegel sowie einen festen Sechs-Grad-Spiegel samt den jeweiligen variierbaren Blenden, womit der Beleuchtungswinkel und die Lichtanteile der jeweiligen Beleuchtungsrichtung variiert werden können. Der Schwerpunkt dieser bekannten Lösung liegt in der Kontraststeigerung mittels einer Koaxialbeleuchtung, wobei es sich bei der Koaxialbeleuchtung um eine achsnahe Schrägbeleuchtung handelt.

In der DE 196 38 263 A1 ist ein ophthalmologisches Beobachtungsgerät offenbart, bei dem der bei Beleuchtung eines Patientenauges zur Beobachtung der vorderen Augenabschnitte unvermeidliche Hornhautreflex unterdrückt werden soll. Dies geschieht durch Anbringen eines Lichtabsorbers in Form eines schwarzen Punkts in der Nähe einer Leuchtfeldblende einer ansonsten bekannten Beleuchtung.

In der US-A-6,011,647 ist ein umschaltbares Beleuchtungssystem für ein ophthalmologisches Operationsmikroskop beschrieben, bei dem zwischen einer Umfeldbeleuchtung und einer optimierten "roter Reflex"-Beleuchtung während der Operation umgeschaltet werden kann. Die Beleuchtungseinrichtung besteht aus Lichtquelle, Kollektor, Leuchtfeldblende, Umlenkspiegel, Feldlinse und Hauptobjektiv. Bei dieser optimierten "roter Reflex"-Beleuchtung wird dann nicht wie bei der Umfeldbeleuchtung die Leuchtfeldblende, sondern die Wendel der Lichtquelle in die Augenpupille als Objektebene abgebildet.

In der EP 1 109 046 A1 ist eine Beleuchtungseinrichtung für ein Operationsmikroskop offenbart, die zwei unabhängig voneinander verschiebbare Reflexionselemente aufweist, mittels derer sowohl die Winkel des einfallenden Lichtes mit der optischen Achse des Mikroskopobjektivs als auch die Intensität der verschiedenen Lichtstrahlen unabhängig voneinander verändert werden können.

Für die Operation am Auge, und hier insbesondere bei Kataraktoperationen, wird ein homogener, heller "roter Reflex" und eine gute Kontrastierung der Phasenobjekte über den gesamten Bereich der Patienten-Augenpupille gefordert.

Die bisher verwendeten Operationsmikroskope erfüllen diese Anforderungen für mehr oder weniger große Bereiche der Augenpupille. Es muss immer ein Kompromiss zwischen den Hauptanforderungen guter, homogener "roter Reflex" und gute Kontrastierung der Phasenobjekte eingegangen werden.

Zumeist wird unter einem kleinen Winkel zur Beobachtung beleuchtet. Dies hat jedoch zur Folge, dass der "rote Reflex" über die Patientenpupille nicht gleichmäßig hell erscheint. Als günstig hat sich bisher ein Beleuchtungswinkel zwischen 2 und 4 Grad bewährt. Bei diesem Winkel erhält man einen guten Kompromiss zwischen guter Kontrastierung und Ausleuchtung der Patientenpupille. Der "rote Reflex" reagiert aber bei dieser Anordnung empfindlich auf ein Verrollen des Patientenauges während der Operation.

Versuche mit einer Koaxial-Beleuchtung führten zwar zu einem guten, homogenen "roten Reflex", aber zu einer schlechten Kontrastierung der Phasenobjekte und haben sich in der Praxis daher bisher nicht bewährt. Dabei war die Beleuchtungsoptik derart angeordnet, dass ein Beleuchtungsspiegel (oder Prisma) zwischen den beiden Strahlengängen des Stereomikroskops lag. Es handelte sich dabei also nicht um eine exakte 0°-Beleuchtung, die genau aus derselben Richtung erfolgt wie die Beobachtung.

In der DE 43 26 761 A1 wird ein Stereomikroskop beschrieben, das eine kontrastreiche Sichtbarmachung transparenter Medien ermöglicht. Dies geschieht durch spezielle Phasenkontrastierungs-Elemente. Im Beleuchtungsstrahlengang kann eine Lichtquellen-Blende vorgesehen sein, die zur Dimensionierung von Größe und/oder Form des gewünschten Lichtquellenbildes auf dem Fundus dient. Die Lichtquellen-Blende kann im Beleuchtungsstrahlengang ein- und ausschwenkbar angeordnet sein.

In der DE 44 17 273 A1 ist schließlich eine Beleuchtungsvorrichtung für Operationsmikroskope beschrieben, bei der das Beleuchtungsstrahlbündel in wenigstens zwei Beleuchtungsteilstrahlbündel aufgeteilt wird, wobei jedes Beleuchtungsteilstrahlbündel des Beleuchtungsstrahls koaxial zu einem Beobachtungsstrahlbündel verläuft. Dadurch soll der "rote Reflex" verbessert werden.

Ausgehend vom genannten Stand der Technik liegt der vorliegenden Erfindung die Aufgabe zugrunde, ein Operationsmikroskop der Eingangs genannten Art weiterzubilden, um die gewünschte Optimierung weiter zu vervollkommnen. Insbesondere soll ein Operationsmikroskop bereitgestellt werden, mit dem eine optimale Problemlösung der praktischen Anforderungen bezüglich Homogenität des "roten Reflexes" und/oder guter Kontrastierung der Linsenreste beziehungsweise Membranen in der Linsenkapsel realisierbar ist.

Diese Aufgabe wird erfindungsgemäß gelöst durch das Operationsmikroskop für die Ophthalmologie mit den Merkmalen des unabhängigen Patentanspruchs 1. Weitere Vorteile, Merkmale, Details, Aspekte und Effekte der Erfindung ergeben sich aus den Unteransprüchen, der Beschreibung sowie den Zeichnungen.

Der Wesensinhalt der vorliegenden Erfindung liegt daher zunächst in einer neuen Konzeption der Beleuchtungseinrichtung. Die neue Konzeption für die Beleuchtungseinrichtung besteht unter anderem darin, dass diese mindestens zwei, aus einer oder auch mehreren Lichtquellen stammende Strahlbündel erzeugt, wobei die optischen Achsen dieser Strahlbündel koaxial zu den optischen Achsen der Beobachtungsstrahlbündel verlaufen.

Beispielsweise kann eine einzige Lichtquelle vorgesehen sein, die zunächst ein einziges Beleuchtungsstrahlbündel erzeugt. Dieses Beleuchtungsstrahlbündel wird anschließend durch geeignete Mittel, beispielsweise Strahlteiler oder dergleichen, in die gewünschte Anzahl von Beleuchtungsteilstrahlbündeln aufgeteilt. Beispielsweise kann aber auch vorgesehen sein, dass die Beleuchtungseinrichtung zwei oder mehr Lichtquellen aufweist, wobei jede Lichtquelle dann ein Beleuchtungsteilstrahlbündel erzeugt.

Es wird somit eine echte koaxiale Beleuchtung geschaffen. Unter "koaxial" wird dabei generell eine achsnahe Beleuchtung verstanden. Dies schließt sowohl eine Beleuchtung unter exakt Null Grad als auch eine achsnahe Schrägbeleuchtung unter einem Winkel von kleiner/gleich 2 Grad mit ein. Das könnte als "im Wesentlichen koaxial" bezeichnet werden. Beispiele hierzu werden im weiteren Verlauf der Beschreibung näher erläutert.

Das neue Beleuchtungskonzept aus mindestens zwei koaxialen Beleuchtungsteilstrahlbündeln erzeugt erfindungsgemäß zwei oder mehrere in der Größe variierbare Beleuchtungsspots auf dem Fundus eines Auges.

Die Erfindung ist nicht auf eine bestimmte Größe der Beleuchtungsspots beschränkt. Die Beleuchtungsspots weisen eine runde Geometrie auf.

Durch eine saubere, beugungsbegrenzte Abbildung der (Sekundär-)Lichtquelle auf den Fundus erhält man dann zusätzlich zu einem homogenen "roten Reflex" auch eine gute Kontrastierung der Phasenobjekte.

Vorteilhaft können die Durchmesser der Beleuchtungsspots in einem Bereich zwischen 0,5 und 1,5 mm auf dem Fundus des Auges variieren. Natürlich können die Beleuchtungsspots auch einen größeren oder kleineren Durchmesser aufweisen. Vorteilhaft kann der Durchmesser des/der Beleuchtungsspots so ausgebildet sein, dass er auf dem Fundus des zu beobachtenden Objekts 1.5 mm, bevorzugt 1.0 mm, weiter bevorzugt 0.5 mm nicht überschreitet.

Die Variation der Beleuchtungsspotdurchmesser wird dabei bedingt durch eine Variation der Beleuchtungsteilstrahlbündel.

Vorteilhaft können die Beleuchtungsstrahlbündel derart ausgebildet sein/werden, dass die Größe der Beleuchtungsspots auf dem Fundus des Auges das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

Die Variation der Beleuchtungsspotdurchmesser geschieht unter Zuhilfenahme von Blenden mit variablen Durchmessern (Irisblenden). Ebenso ist es möglich, die Variation über ein geeignetes Zoom-System zu realisieren. Die letztgenannte Variante hat den weiteren Vorteil, das die Lichtintensität im Beleuchtungsspot zunimmt, wenn der Durchmesser des Beleuchtungsspots verkleinert wird.

Durch die Variation der Beleuchtungsspots auf dem Fundus eines Auges können die Intensität (Helligkeit) und die Homogenität des "roten Reflexes" beeinflusst werden. Je größer der Durchmesser des Beleuchtungsspots gewählt ist, desto homogener und heller ist der "rote Reflex". Je kleiner der Durchmesser des Beleuchtungsspots gewählt ist, desto besser ist die Kontrastierung des "roten Reflexes". Der geeignete Durchmesser eines Beleuchtungsspots ist nunmehr, je nach Bedarf und Anwendungsfall, frei einstellbar.

Mit der Beleuchtungseinrichtung wird eine exakte koaxiale Beleuchtung realisiert, die einen homogenen "roten Reflex" liefert, und die darüber hinaus auch noch gegen ein Verrollen des Patientenauges unempfindlich ist. Dadurch kann auch eine eventuelle Nachführung der Beleuchtung zur Optimierung des "roten Reflexes" bei verrolltem Auge entfallen, wodurch sich der Aufbau der Beleuchtungseinrichtung beziehungsweise eines entsprechenden Operationsmikroskops vereinfacht.

Erfindungsgemäß ist jedes Beleuchtungsteilstrahlbündel derart geführt, dass ein Auge bezüglich jedes Beobachtungsstrahlbündels aus derselben Richtung beleuchtet ist, aus der auch die Beobachtung erfolgt (0°-Beobachtung). Jedes Beleuchtungsteilstrahlbündel wird derart geführt, dass für den linken und rechten Beobachtungsstrahlengang des (Stereo-)Operationsmikroskops das Auge aus derselben Richtung beleuchtet wird, aus der auch die Beobachtung erfolgt. Es liegt somit eine exakte 0°-Beleuchtung für jeden Beobachtungsstrahlengang vor.

Gemäß einer anderen erfindungsgemäßen Ausgestaltung ist jedes Beleuchtungsteilstrahlbündel derart geführt, dass ein Auge bezüglich jedes Beobachtungsstrahlbündels in einem Winkel von kleiner/gleich 2 Grad, vorzugsweise kleiner/gleich 1 Grad schräg beleuchtet ist/wird (achsnahe Schrägbeleuchtung). Das Auge wird somit in einem kleinen Winkel zur Beobachtung beleuchtet.

Durch eine saubere, beugungsbegrenzte Strahlführung für die Beleuchtung und kleine Beleuchtungsspots auf dem Fundus eines Patientenauges erhält man einen optimalen "roten Reflex" bei gleichzeitig guter Kontrastierung. Ferner reagiert diese Beleuchtungseinrichtung sehr unkritisch auf ein Verrollen des Patientenauges während der Operation.

In weiterer Ausgestaltung ist vorteilhaft vorgesehen, dass die Entfernung der Mitte des Beleuchtungsspots von der Mitte der Querschnittsfläche des Beobachtungsstrahlbündels auf dem Fundus das 0.8-fache, bevorzugt das 0.5-fache, weiter bevorzugt das 0.2-fache, besonders bevorzugt das 0.05-fache des Radius der Querschnittsfläche des Beobachtungsstrahlbündels auf dem Fundus beträgt.

Durch die Beleuchtungseinrichtung eines Operationsmikroskops gemäß der vorliegenden Erfindung kann insbesondere erreicht werden, dass sich die optimale Größe des Beleuchtungsspots nach der Fehlsichtigkeit des Patienten und der Vergrößerung des Operationsmikroskops richtet. Die wird beispielsweise durch die Relativangaben von Beleuchtungsspotgröße zu Querschnittsfläche der Beobachtungsstrahlenkegel auf dem Fundus erreicht. Es werden die wesentlichen Merkmale für einen optimalen Rotreflex verwirklicht, nämlich kleine Spotgröße für guten Kontrast sowie die Lage des Beleuchtungsspots auf dem Fundus.

Vorteilhaft kann die Beleuchtungseinrichtung wenigstens ein Objektivelement aufweisen. Das Objektivelement kann dabei ebenfalls als Objektivelement des Operationsmikroskops, insbesondere als dessen Hauptobjektiv, ausgebildet sein. Dies ist jedoch nicht zwingend erforderlich.

Weiterhin können in der Beleuchtungseinrichtung verschiedene optische Elemente vorgesehen sein, die zwischen der wenigstens einen Lichtquelle und dem wenigstens einen Objektivelement angeordnet sind.

In vorteilhafter Ausgestaltung sind Mittel vorgesehen, um jeweils ein Beobachtungsstrahlbündel und ein Beleuchtungsteilstrahlbündel beziehungsweise das Beleuchtungsstrahlbündel zu überlagern. Diese Mittel können auf unterschiedlichste Weise ausgestaltet und an unterschiedlichsten Orten angeordnet sein. Nachfolgend werden hierzu einige nicht ausschließliche Beispiele erläutert.

Beispielsweise kann vorgesehen sein, dass die Mittel zum Überlagern derart angeordnet sind, dass eine Überlagerung von Beobachtungsstrahlbündel und Beleuchtungsteilstrahlbündel beziehungsweise Beleuchtungsstrahlbündel oberhalb des Objektivelements erfolgt. Die Überlagerung von Beobachtungsstrahlbündel und Beleuchtungsteilstrahlbündel beziehungsweise Beleuchtungsstrahlbündel kann beispielsweise im parallelen Strahlengang über dem Hauptobjektiv erfolgen.

Beispielsweise kann auch vorgesehen sein, dass die Mittel zum Überlagern derart angeordnet sind, dass eine Überlagerung von Beobachtungsstrahlbündel und Beleuchtungsteilstrahlbündel beziehungsweise Beleuchtungsstrahlbündel unterhalb des Objektivelements erfolgt. Es besteht somit auch die Möglichkeit, die Beleuchtungsteilstrahlbündel beziehungsweise das Beleuchtungsstrahlbündel und die Beobachtungsstrahlbündel unterhalb des Hauptobjektivs zu überlagern. In diesem Fall ist es vorteilhaft, wenn die Beleuchtungsteilstrahlbündel entsprechend der Hauptobjektivbrennweite geneigt werden.

Vorteilhaft kann insbesondere im letztgenannten Fall vorgesehen sein, dass das Objektivelement als so genannte Varioskopoptik ausgebildet ist. Bei einer Varioskopoptik handelt es sich generell um eine Optik mit wenigstens zwei durch einen Abstand getrennten optischen Elementen, wobei durch Variation dieses Abstands der freie Arbeitsabstand zwischen Objektiv und Objektebene variiert werden kann. Eine solche Varioskopoptik an sich ist bereits aus dem Stand der Technik bekannt. In dem zuvor beschriebenen Fall mit Überlagerung der Strahlbündel unterhalb des Objektivelements ist es bei Verwendung einer Varioskopoptik vorteilhaft, wenn die Beleuchtungsteilstrahlbündel entsprechend dem freien Arbeitsabstand nachgeführt werden.

Wie oben schon ausgeführt wurde, ist die Erfindung nicht auf bestimmte Ausgestaltungstypen von "Überlagerungsmitteln" beschränkt. Beispielsweise können die Mittel zum Überlagern wenigstens ein optisches Element in Form eines Prismas und/oder einer Strahlteilerplatte und/oder eines Spiegels, etwa eines teildurchlässigen Spiegels und/oder eines durchbohrten Spiegels, aufweisen. Natürlich können die Mittel auch anders ausgestaltet sein, so dass die Erfindung nicht auf die genannten Beispiele beschränkt ist.

Erfindungsgemäß ist eine Vorrichtung zum Verändern des Bündelquerschnitts des wenigstens eines Beleuchtungsstrahls und/oder wenigstens eines Beleuchtungsteilstrahlbündels vorgesehen. Die Vorrichtung ist als Irisblende ausgebildet. Durch den Einbau einer entsprechenden Vorrichtung in der Beleuchtungseinrichtung, etwa im Beleuchtungsstrahl, ist es möglich, den Leuchtfleck auf dem Fundus des Patientenauges zu variieren. Ein kleiner Lichtfleck liefert einen besseren Kontrast. Bei Kataraktoperationen kann der Fall auftreten, dass insbesondere bei dichten Katarakten der "rote Reflex" zu dunkel erscheinen kann. Dann ist es von Vorteil, den Leuchtfleck und damit die Helligkeit zu vergrößern. Die Intensität der Strahlung auf der Retina wird dadurch nicht vergrößert. Negative Auswirkungen auf den Kontrast sind nicht zu erwarten, da bei einem sehr dichten Katarakt der Leuchtfleck ohnehin aufgestreut wird.

Ein weiterer Vorteil der Beleuchtungseinrichtung besteht darin, dass auf der Hornhaut-Vorderfläche des Patientenauges nur ein Hornhautreflex sichtbar wird, da sich die Beleuchtungsteilstrahlbündel an dieser Stelle annähernd überdecken.

In einer vorteilhaften Ausgestaltung kann vorgesehen sein, dass zwei oder mehr Lichtquellen vorgesehen sind und dass mittels jeder Lichtquelle ein Beleuchtungsteilstrahlbündel erzeugt wird. Es können somit unabhängige Lichtquellen verwendet werden, wobei jede Lichtquelle ein eigenes Beleuchtungsteilstrahlbündel erzeugt.

In einer anderen vorteilhaften Ausgestaltung kann vorgesehen sein, dass eine einzige Lichtquelle vorgesehen ist und dass Mittel zum Aufteilen des Beleuchtungsstrahlbündels der Lichtquelle in die zwei oder mehr Beleuchtungsteilstrahlbündel vorgesehen sind. Hierbei kann es sich um geeignete Strahlteiler in Form von Prismen, teildurchlässigen Spiegeln und dergleichen handeln.

Die vorliegende Erfindung ist nicht auf die Verwendung bestimmter Lichtquellen beschränkt. Nachfolgend werden hierzu einige nicht ausschließliche, vorteilhafte Beispiele genannt. Beispielsweise kann die wenigstens eine Lichtquelle als Lampe, insbesondere als Halogenlampe oder Xenonlampe, als Laser, als nicht thermischer Strahler, als Lichtleiter, insbesondere als Faserlichtleiterbündel, als wenigstens eine LED (Licht emittierende Diode), als wenigstens eine OLED (organische Licht emittierende Diode) oder dergleichen ausgebildet sein. Natürlich sind auch Kombinationen verschiedener Lichtquellen möglich.

Vorteilhaft kann die Lichtquelle aus einer Anordnung von einer oder mehreren Leuchtdiode(n) (LED), insbesondere organischen Leuchtdiode(n) (OLED), gebildet sein. Organische Leuchtdioden sind ursprünglich als Mikrodisplays entwickelt worden. Anders als LCDs, die eine Hinterleuchtung benötigen, leuchten OLEDs selber als Lambertstrahler (Flächenemitter).

Als strukturierte Beleuchtungsquelle bieten OLEDs eine gute Lichteffizienz und kleine Strukturen ohne dunkle Zwischenräume. Entsprechend einer gewünschten Beleuchtungsgeometrie können einzelne der Kleinstlichtquellen angeschaltet werden und andere ausgeschaltet bleiben. Gegenüber LEDs ist bei OLEDs der Füllfaktor höher was bedeutet, dass eine höhere Packungsdichte realisierbar ist. Die Verwendung eines Displays aus LEDs oder OLEDs ermöglicht ein programmierbares, und beispielsweise auch automatisierbares Schalten unterschiedlicher Beleuchtungsmodi, ohne dass mechanische Komponenten, wie etwa Phasenkontrastringe, Filter, Abschwächer und dergleichen bewegt werden müssten. Besonders geeignet sind beispielsweise weiße OLEDs, deren Spektrum durch eine Mischung von organischen Molekülen bestimmt wird.

Zusammenfassend weist das wie vorstehend beschriebene Operationsmikroskop eine ganze Reihe von Vorteilen auf. Durch eine koaxiale Beleuchtung, insbesondere durch eine "echte" 0°-Beleuchtung, lässt sich ein sehr homogener und heller "roter Reflex" erzeugen. Der "rote Reflex" reagiert auf ein Verkippen des Patientenauges sehr unempfindlich. Das heißt, auf eine Nachführung bezüglich Winkeln kann verzichtet werden. Über die Integration einer Vorrichtung zum Verändern des Strahlbündelquerschnitts, beispielsweise einer (Doppel-)Irisblende kann die Helligkeit des "roten Reflexes" und der Kontrast von Phasenstrukturen an die Behandlungssituation angepasst und optimiert werden. Durch die Verringerung des Irisblendendurchmessers wird der Kontrast verbessert, allerdings nimmt auch die Helligkeit ab.

Das Operationsmikroskop kann beispielsweise ein Hauptobjektivelement aufweisen, welches identisch mit einem Objektivelement der Beleuchtungseinrichtung ist. Weiterhin können Mittel vorgesehen sein, um jeweils ein Beobachtungsstrahlbündel und ein Beleuchtungsteilstrahlbündel beziehungsweise Beleuchtungsstrahlbündel zu überlagern. Die Mittel zum Überlagern können derart angeordnet sein, dass eine Überlagerung von Beobachtungsstrahlbündel und Beleuchtungsteilstrahlbündel beziehungsweise Beleuchtungsstrahlbündel oberhalb des Hauptobjektivelements erfolgt.

In anderer Ausgestaltung kann vorgesehen sein, dass das Operationsmikroskop ein Hauptobjektivelement aufweist, welches identisch mit einem Objektivelement der Beleuchtungseinrichtung ist, dass Mittel vorgesehen sind, um jeweils ein Beobachtungsstrahlbündel und ein Beleuchtungsteilstrahlbündel beziehungsweise Beleuchtungsstrahlbündel zu überlagern und dass die Mittel zum Überlagern derart angeordnet sind, dass eine Überlagerung von Beobachtungsstrahlbündel und Beleuchtungsteilstrahlbündel beziehungsweise Beleuchtungsstrahlbündel unterhalb des Hauptobjektivelements erfolgt.

Für den letztgenannten Fall kann vorteilhaft vorgesehen sein, dass das Hauptobjektivelement als so genannte Varioskopoptik ausgebildet ist. Zu der Ausgestaltung und Funktionsweise der Varioskopoptik wird auf die entsprechenden Ausführungen weiter oben im Zusammenhang mit der erfindungsgemäßen Beleuchtungseinrichtung verwiesen.

Vorteilhaft kann das Operationsmikroskop als Stereomikroskop ausgebildet sein. Das optische System eines Operationsmikroskops besteht grundsätzlich aus mehreren Bauelementen, wie dem Tubus, dem Mikroskop-Grundkörper, usw. Zusätzlich ist es bei vielen Operationsmikroskopen möglich, unterschiedliche Zusatzmodule, wie zum Beispiel einen Mitbeobachtertubus für einen assistierenden Beobachter, eine Videokamera zur Dokumentation oder dergleichen anzuschließen.

Innerhalb des Mikroskop-Grundkörpers lassen sich wiederum mehrere Baugruppen zusammenfassen, wie beispielsweise eine Beleuchtungseinrichtung, eine Vergrößerungseinrichtung, das Hauptobjektiv oder dergleichen. Die charakteristische Größe beim Hauptobjektiv ist seine Brennweite, die den Arbeitsabstand vom Operationsmikroskop zum Operationsfeld festlegt und auch Einfluss auf die Gesamtvergrößerung des Mikroskops hat.

Vorzugsweise kann in dem wenigstens einen Beobachtungsstrahlengang ein Vergrößerungssystem vorgesehen sein. Hierbei kann es sich beispielsweise um einen Vergrößerungswechsler handeln, mit dem sich unterschiedliche Vergrößerungen einstellen lassen. In vielen Anwendungsfällen ist ein Vergrößerungswechsel in Stufen völlig ausreichend. Es ist jedoch auch möglich, als Vergrößerungssystem auch pankratische Vergrößerungssysteme zu verwenden, mittels derer eine stufenlose Vergrößerung (Zoomsystem) möglich ist.

Dabei kann vorteilhaft vorgesehen sein, dass die weiter oben bereits beschriebene Gerätepupille der Beobachtungseinrichtung in dem Vergrößerungssystem angesiedelt ist.

Weiterhin können in dem wenigstens einen Beobachtungsstrahlengang ein Tubuselement und ein Okularelement vorgesehen sein. Die Aufgabe eines Okularelements ist generell die Nachvergrößerung des im Tubus entstehenden Zwischenbilds, sowie möglicherweise der Ausgleich eventueller Fehlsichtigkeiten des Nutzers eines solchen Mikroskops.

Vorteilhaft ist weiterhin vorgesehen, dass die Objektebene des Auges im vorderen Brennpunkt des Hauptobjektivs ausgebildet ist. Dadurch wird erreicht, dass das Auge durch das Hauptobjektiv nach Unendlich abgebildet wird.

Bei dem Operationsmikroskop kann es sich gemäß einer bevorzugten Ausführungsform um ein Stereomikroskop nach dem Teleskopprinzip handeln, das im Wesentlichen aus den drei optischen Teilkomponenten, nämlich Hauptobjektiv (afokales) Zoomsystem sowie binokulares Fernrohr aus Tubus und Okularen, besteht.

Zwischen den einzelnen Teilkomponenten des Operationsmikroskops verlaufen die Beobachtungsstrahlenbündel vorzugsweise parallel, sodass die einzelnen Teilkomponenten modular austauschbar und kombinierbar sind.

Gemäß der vorliegenden Erfindung sind insbesondere die prinzipiellen Grundanforderungen für ein optimiertes Beleuchtungssystem in der Kataraktchirurgie festgelegt worden, nämlich eine koaxiale Beleuchtung für die Homogenität des roten Reflexes eine stigmatische, beugungsbegrenzte Abbildung der wohldefinierten Beleuchtungsspots für die gute Kontrastierung des Roten Reflexes.

Für die Erzeugung der koaxialen Beleuchtungsstrahlenbündel wird beispielsweise ein Prismensystem vorgeschlagen.

Darüber hinaus gestattet das erfindungsgemäße Operationsmikroskop eine einfache Umschaltung zwischen der optimierten Red Reflex - Beleuchtung und der für die praktische Anwendung unabdingbaren Umfeldbeleuchtung zur vollständigen Ausleuchtung des maximalen Sehfelds bei der stereoskopischen Beobachtung.

Die Erfindung wird nun anhand von Ausführungsbeispielen unter Bezugnahme auf die beiliegenden Zeichnungen näher erläutert. Es zeigen
- Figur 1: in schematischer Darstellung eine mögliche Anordnung zur Erzeugung einer 0°-Beleuchtung, bei gleichzeitig optimalem "roten Reflex" und guter Kontrastierung;
- Figur 2: in schematischer Darstellung den Aufbau eines optischen Systems für die Rotreflex-Beleuchtung;
- Figur 3: in schematischer Darstellung den Aufbau eines optischen Systems für die Umfeldbeleuchtung; und
- Figur 4: in schematischer Darstellung den Aufbau einer vorteilhaften Aperturblende, wie sie in den Beleuchtungseinrichtung gemäß Figur 2 eingesetzt ist.

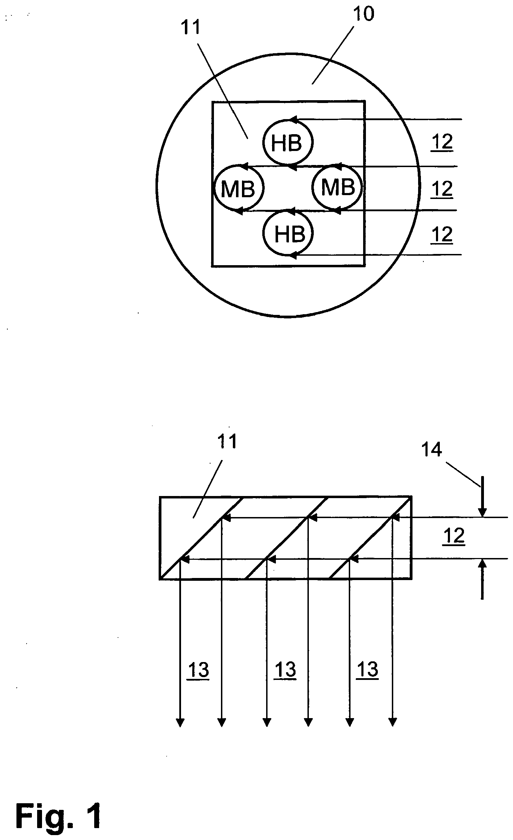

In Figur 1 sind Teile einer Beleuchtungseinrichtung dargestellt, die in einem Operationsmikroskop eingesetzt ist. Bei dem Operationsmikroskop handelt es sich um ein Stereo-Operationsmikroskop zum Einsatz in der Ophthalmo-Chirurgie, beispielsweise zur Durchführung von Kataraktoperationen. Mittels der Beleuchtungseinrichtung wird ein sehr gleichmäßiger, heller "roter Reflex" dadurch erzielt, dass das Beleuchtungsstrahlbündel 12 in mehrere Beleuchtungsteilstrahlbündel 13 aufgeteilt wird. Dies geschieht durch Mittel 11 zum Aufteilen des Beleuchtungsstrahlbündels, welche hierfür über eine geeignete Spiegel/Prismenanordnung verfügen können. Die Beleuchtungsteilstrahlbündel 13 sind dabei so geführt, dass bezüglich des linken und rechten Beobachtungsstrahlengangs des Operationsmikroskops das zu beobachtende Objekt, im vorliegenden Fall ein Patientenauge, aus derselben Richtung beleuchtet wird, aus der auch die Beobachtung erfolgt (0°-Beleuchtung).

Wie dem linken Teil der Figur zu entnehmen ist, sind bei dem dargestellten Beispiel Beobachtungssstrahlengänge sowohl für einen Hauptbeobachter (HB) als auch für einen Mitbeobachter (MB) vorgesehen. Die Mittel 11 zur Aufteilung des Beleuchtungsstrahlengangs können beispielsweise im Bereich eines Objektivelements 10 angeordnet sein, bei dem es sich beispielsweise auch um das Hauptobjektiv der Beobachtungseinrichtung handeln kann.

Durch eine saubere, beugungsbegrenzte Strahlführung für die Beleuchtung und kleine Beleuchtungsspots auf dem Fundus des Patientenauges (Durchmesser etwa 0,5 bis 1,5mm) erhält man mit dieser Anordnung einen optimalen "roten Reflex" bei gleichzeitig guter Kontrastierung. Ferner reagiert diese Beleuchtungsanordnung sehr unkritisch auf ein Verrollen des Patientenauges während der Operation.

Die Überlagerung von Beobachtungsstrahlbündel und Beleuchtungsteilstrahlbündel 13 kann beispielsweise im parallelen Strahlengang über dem Objektivelement 10 (dem Hauptobjektiv) durch die Mittel 11 erfolgen, bei denen es sich vorteilhaft um teildurchlässige Spiegel oder Prismen handelt.

Ferner ist in der Beleuchtungseinrichtung eine Vorrichtung 14 zum Verändern der Strahlbündelquerschnitte in Form einer Irisblende vorgesehen. Dadurch kann im Beleuchtungsstrahl 12 der Leuchtfleck auf dem Fundus des Patientenauges variiert werden.

Im vorliegenden Beispiel wird das Beleuchtungsstrahlbündel 12 von einer einzigen Lichtquelle (nicht dargestellt) erzeugt und über die Mittel 11 auf mehrere Beleuchtungsteilstrahlbündel 13 aufgeteilt. Es ist jedoch auch denkbar, mehrere voneinander unabhängige Lichtquellen zu verwenden, wobei jede Lichtquelle jeweils mindestens ein Beleuchtungsteilstrahlbündel 13 erzeugt.

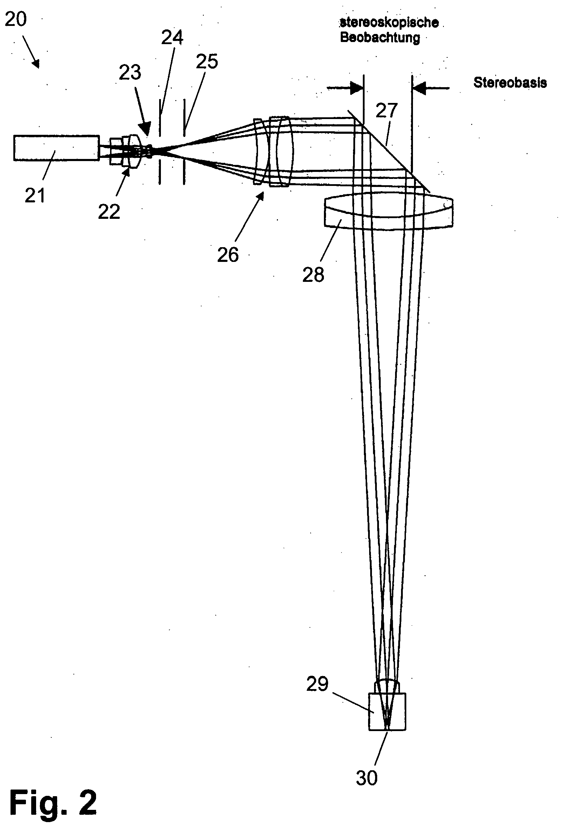

Der Aufbau des optischen Systems für die Rotreflex - Beleuchtung ist in Figur 2 skizziert. Von einer Lichtquelle aus betrachtet werden in der dargestellten Beleuchtungseinrichtung 20 folgende optischen Komponenten verwendet: ein Lichtleiter 21, ein Kollektor 22, eine Plankonvexlinse 23, eine Leuchtfeldblende 24, eine Aperturblende (Lochblende) 25, eine optische Komponente 26, beispielsweise eine Teilkomponente aus Kittglied und Meniskus, ein Umlenkelement 27, beispielsweise in Form eines Teilerspiegels sowie ein Objektivelement 28, beispielsweise in Form eines Hauptobjektivs. Beleuchtet werden soll ein Auge 29 mit Fundus 30.

Vom Faserende des Lichtleiters 21 wird mit einem Kollektor 22 und einer Plankonvexlinse 23 ein reelles Zwischenbild erzeugt. Am Ort dieses Zwischenbilds kann eine Aperturblende 25 z.B. in Form einer Lochblende angebracht werden. Dieses reelle Zwischenbild liegt in der vorderen Brennebene einer zweigliedrigen Teiloptik bestehend aus dem Hauptobjektiv 28 und der Teilkomponente 26 aus Kittglied mit Meniskuslinse. Diese Teiloptik bildet dann ein weiteres virtuelles Zwischenbild im Unendlichen, so dass vom Auge 29 aus betrachtet das Faserende des Lichtleiters 21 im Fernpunkt liegt. Infolgedessen wird dann bei einem rechtsichtigen Auge das Faserende des Lichtleiters 21 als Beleuchtungsspot auf den Fundus 30 abgebildet.

Die wirksame Leuchtfläche des Faserendes vom Lichtleiter 21 kann beispielsweise 4.8 mm betragen. Der Durchmesser des Zwischenbilds in der Aperturblende 25 beträgt dann 5.8 mm. Für die Größe des Beleuchtungsspots auf dem Fundus 30 erhält man in dem genannten Beispiel einen Durchmesser von 1.5 mm.

Mittels Lochblenden wohldefinierter Lage können in der Zwischenbildebene (Aperturblende) die für die gute Homogenität des Roten Reflexes erforderlichen zu den stereoskopischen Beobachtungsachsen koaxialen Strahlenbüschel im Abstand der Stereobasis der stereoskopischen Beobachtungsachsen erzeugt werden. Die Lochblenden werden bezüglich Lage, also Lateralversatz zur optischen Achse, und Größe, also Durchmesser der Lochblenden, mit demselben Abbildungsmaßstab wie das reelle Zwischenbild des Faserendes, nämlich 5.8 : 1.5 = 3.9 : 1, verkleinert auf den Fundus abgebildet. Die Grösse des Durchmessers der Lochblenden bestimmt dann die Größe der Beleuchtungsspots auf dem Fundus und somit in entscheidender Weise die gute Kontrastierung des Roten Reflexes.

Zwischen der Plankonvexlinse 23 und dem reellen Zwischenbild des Faserendes (Aperturblende) befindet sich die Leuchtfeldblende 24. Diese Leuchtfeldblende 24 dient zur Begrenzung des ausgeleuchteten Sehfelds.

Die Leuchtfeldblende 24 liegt im vorderen Brennpunkt der Teilkomponente 26 aus Kittglied und Meniskuslinse. Die Leuchtfeldblende 24 wird also durch die Teilkomponente 26 zunächst virtuell nach Unendlich und schließlich mit dem Hauptobjektiv 28 auf die in der vorderen Brennebene des Hauptobjektivs sich befindliche Objektebene abgebildet.

Der Durchmesser der Leuchtfeldblende 24 beträgt beispielsweise 2.5 mm. Dies führt zu einem ausgeleuchteten Sehfeld in der Objektebene von 10 mm. Somit beträgt der Abbildungsmaßstab für die Leuchtfeldabbildung 1 : 4.

In der Tabelle 1 sind die optischen Systemdaten für die Rotreflex - Beleuchtung aufgelistet:

### Systemdaten für Red Reflex - Beleuchtungssystem

**Tabelle 1**

| **Nr.** | **Radius** (mm) | **Dicke** (mm) **Lichtleiter** | **Medium** | **freier Durchmesser** (mm) |
|---|---|---|---|---|
| | | 4.7 | Luft | |
| 1 | -49.759 | 5.0 | NSK2 | 8.5 |
| 2 | -17.655 | 0.1 | Luft | 10.8 |
| 3 | -37.047 | 2.0 | NSF6 | 11.0 |
| 4 | 26.227 | 5.5 | NSK2 | 12.2 |
| 5 | -12.589 | 2.0 | Luft | 13.6 |
| 6 | 6.6355 | 2.0 | NSK2 | 5.0 |
| 7 | Plan | 1.9 | Luft | 5.0 |
| 8 | Plan | 13.1 | Luft | **Blende** |
| 9 | Plan | 29.2 | Luft | **Blende** |
| 10 | -58.294 | 5.0 | NSK2 | 26.0 |
| 11 | -28.387 | 0.1 | Luft | 27.0 |
| 12 | 392.42 | 3.0 | NSF6 | 28.0 |
| 13 | 45.316 | 7.0 | NSK2 | 29.0 |
| 14 | -55.033 | 40.0 | Luft | 29.0 |
| 15 | Plan | 17.0 | Luft | Spiegel |
| 16 | 120.57 | 10.5 | NFK51 | 53.0 |
| 17 | -79.719 | 5.1 | NBAF4 | 53.0 |
| 18 | -244.06 | 188.3 | Luft | 53.0 |
| 19 | 8.0 | 6.0 | BAK4 | |
| 20 | Plan | 15.4 | BK7 | **Modellauge** |
| | | **Fundus** | | |

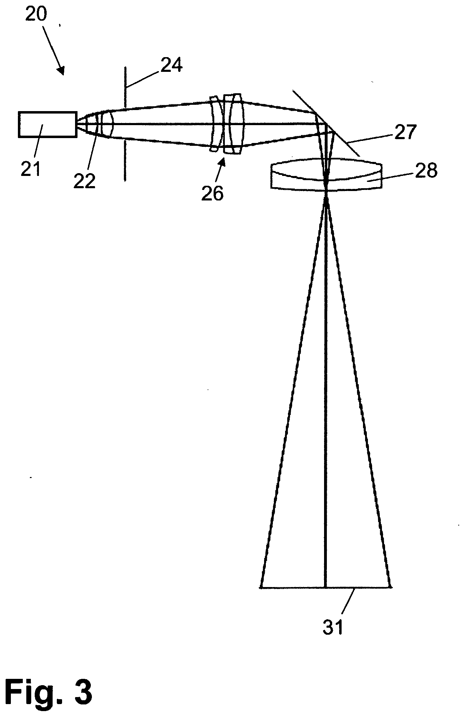

Der Aufbau des optischen Systems für die Umfeldbeleuchtung ist in Figur 3 skizziert.

Ein wesentlicher Gedanke besteht darin, dass die Umfeld - Beleuchtung ohne zusätzliche optische Komponenten durch einen einfachen Umschaltvorgang aus der Rotreflex - Beleuchtung abgeleitet werden kann.

Die für die Umfeld - Beleuchtung notwendigen optischen Komponenten sind also bis auf die Größe der Leuchtfeldblende identisch mit den optischen Komponenten der Rotreflex Beleuchtung gemäß Figur 2, nämlich: Lichtleiter 21, Kollektor 22, Leuchtfeldblende 24, optische Komponente 26, beispielsweise in Form einer Teilkomponente aus Kittglied und Meniskus, Umlenkelement 27, beispielsweise in Form eines Teilerspiegels, sowie Objektivelement 28, beispielsweise in Form eines Hauptobjektivs.

Bei der Umschaltung von der Rotreflex - Beleuchtung zur Umfeldbeleuchtung wird die Plankonvexlinse und die Lochblende (siehe Figur 2) herausgeschwenkt. Des Weiteren wird eine kleine Leuchtfeldblende durch eine große Leuchtfeldblende 24 ersetzt.

Mit dem Kollektor 22 wird nun die Leuchtfeldblende 24 vollständig ausgeleuchtet. Die Leuchtfeldblende 24 sitzt ja wie bei der Rotreflex Beleuchtung unverändert in der vorderen Brennebene der Teiloptik 26 aus Kittglied und Meniskuslinse. Die Leuchtfeldblende 24 wird also virtuell nach Unendlich abgebildet, so dass das Bild der Leuchtfeldblende 24 wie bei der Rotreflex - Beleuchtung durch die Abbildung mit dem Hauptobjektiv 28 wiederum in der vorderen Fokusebene des Hauptobjektivs 28 und somit in der Objektebene der Beobachtung liegt.

Der Durchmesser der Leuchtfeldblende 24 beträgt beispielsweise 14 mm. Damit kann man eine Ausleuchtung des maximalen Sehfelds 31 in der Objektebene von ca. 62 mm erreichen. Die Vergrößerung des Abbildungsmaßstabs gegenüber der Rotreflex Beleuchtung ist durch die Verzeichnung der Leuchtfeldabbildung erklärbar.

Der optische Aufbau für die hier vorgeschlagene Rotreflex Beleuchtung ermöglicht einen unabhängigen Eingriff in die Strahlengänge für die Pupillen- und Leuchtfeldabbildung. So kann beispielsweise die Lichtausbeute optimal an die Leuchtfeldgröße angepasst und die Größe der Beleuchtungsspots auf dem Fundus durch gezielten Eingriff in der Aperturblende angepasst werden.

Bei der Umfeld - Beleuchtung reduziert sich die optische Problemstellung auf die Abbildung der optimal ausgeleuchteten Leuchtfeldblende.

Für die Pupillenabbildung ergibt sich aufgrund des optischen Aufbaus zwangsläufig ein reelles Zwischenbild des Faserendes in der Nähe der Frontfläche des Hauptobjektivs. Üblicherweise liegt auch bei den derzeit eingesetzten Beleuchtungen für die Ophthalmologie dieses reelle Zwischenbild im Objektraum, und zwar ca. 50 mm unter dem Hauptobjektiv.

Die optischen Systemdaten für die Umfeld - Beleuchtung sind in Tabelle 2 aufgelistet:

### Systemdaten für Umfeld - Beleuchtungssystem

**Tabelle 2**

| **Nr.** | **Radius** (mm) | **Dicke** (mm) **Lichtleiter** | **Medium** | **freier Durchmesser** (mm) |
|---|---|---|---|---|
| 1 | -49.759 | 4.7 | Luft | 8.5 |
| 2 | -17.655 | 5.0 | NSK2 | 10.8 |
| 3 | -37.047 | 0.1 | Luft | 11.0 |
| 4 | 26.227 | 2.0 | NSF6 | 12.2 |
| 5 | -12.589 | 5.5 | NSK2 | 13.6 |
| 6 | Plan | 5.9 | Luft | **Blende** |
| 7 | -58.294 | 42.3 | Luft | 26.0 |
| 8 | -28.387 | 5.0 | NSK2 | 27.0 |
| 9 | 392.42 | 0.1 | Luft | 28.0 |
| 10 | 45.316 | 3.0 | NSF6 | 29.0 |
| 11 | -55.033 | 7.0 | NSK2 | 29.0 |
| 12 | Plan | 40.0 | Luft | Spiegel |
| 13 | 120.57 | 17.0 | Luft | 53.0 |
| 14 | -79.719 | 10.5 | NFK51 | 53.0 |
| 15 | -244.06 | 5.1 | NBAF4 | 53.0 |
| | | 193.6 | Luft | |
| | | **Sehfeld** | | |

Es kann durchaus auch sinnvoll sein, dem Anwender gleichzeitig eine Rotreflex- und eine Umfeldbeleuchtung zur Verfügung zu stellen. Dies kann beispielsweise dadurch erfolgen, dass die Plankonvexlinse 23 zwischen dem Kollektor 22 und den Blenden 24, 25 entweder auf einen transparenten Träger aufgekittet wird, oder beispielsweise als Kunststoffspritzgussteil (PMMA) mit einem entsprechenden transparentem Tragrand ausgebildet wird. Die Strahlen, welche die Plankonvexlinse 23 durchsetzen, erzeugen die Rotreflex Beleuchtung, die Strahlen die den Träger oder Tragrand durchsetzten, erzeugen die Umfeldbeleuchtung.

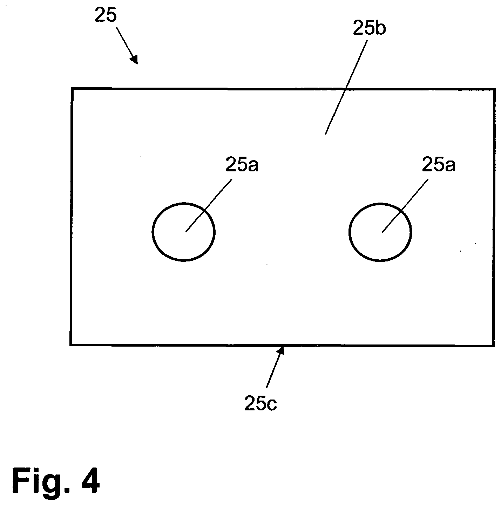

Um gleichzeitig die Rotreflex- und die Umfeldbeleuchtung zu erzeugen ist es ferner vorteilhaft, die Aperturblende 25 (Lochblende) in besonderer Weise zu gestalten. Ein Beispiel hierzu ist in Figur 4 dargestellt.

Gemäß Figur 4 kann die Aperturblende 25 vorteilhaft derart ausgestaltet sein, dass die Öffnungen 25a für die Rotreflexbeleuchtung beispielsweise eine hohe Transmission und die umliegenden Bereich 25b eine reduzierte (im Idealfall einstellbare) Transmission für die Umfeldbeleuchtung aufweisen.

Realisiert werden kann dies beispielsweise über ein transmissives oder reflektives LCD Display 25c, beziehungsweise über ein DMD Display.

### Bezugszeichenliste

- 10: Objektivelement
- 11: Mittel zum Aufteilen des Beleuchtungsstrahls
- 12: Beleuchtungsstrahlbündel
- 13: Beleuchtungsteilstrahlbündel
- 14: Vorrichtung zum Verändern des Strahlbündelquerschnitts

- 20: Beleuchtungseinrichtung
- 21: Lichtleiter
- 22: Kollektor
- 23: Plankonvexlinse
- 24: Leuchtfeldblende
- 25: Aperturblende
- 25a: Öffnung
- 25b: umliegender Bereich
- 25c: Display
- 26: optische Komponente
- 27: Umlenkelement
- 28: Objektivelement
- 29: Auge
- 30: Fundus
- 31: Sehfeld

## Patentansprüche

1. Operationsmikroskop für die Ophthalmologie, mit einem, zwei oder mehr stereoskopischen Beobachtungsstrahlengängen mit jeweils einem Beobachtungsstrahlbündel und mit einer Beleuchtungseinrichtung, aufweisend zumindest eine Lichtquelle zum Erzeugen wenigstens eines Beleuchtungsstrahls (12) zum Beleuchten eines zu beobachtenden Auges, wobei wenigstens zwei Beleuchtungsteilstrahlbündel (13) vorgesehen sind und wobei jedes Beleuchtungsteilstrahlbündel (13) koaxial, das heißt unter exakt Null Grad oder in Form einer achsnahen Schrägbeleuchtung unter einem Winkel von kleiner/gleich 2 Grad zu einem stereoskopischen Beobachtungsstrahlbündel verläuft, wobei wenigstens eine Vorrichtung (14) zum Verändern des Bündelquerschnitts wenigstens eines Beleuchtungsteilstrahlbündels (13) vorgesehen ist, wobei die Vorrichtung (14) als Irisblende oder als Zoom-System ausgebildet ist, und wobei die Vorrichtung (14) ausgebildet ist, dass die Beleuchtungsteilstrahlbündel (13) derart ausgebildet sind, dass sie zwei oder mehrere in der Größe variierbare Beleuchtungsspots auf dem Fundus eines zu beobachtenden Auges bilden, wobei die Beleuchtungsspots eine runde Geometrie aufweisen, und wobei die Durchmesser der Beleuchtungsspots einstellbar sind.

2. Operationsmikroskop nach Anspruch 1, **dadurch gekennzeichnet, dass** die Beleuchtungsteilstrahlbündel (13) derart ausgebildet sind, dass die Größe der Beleuchtungsspots auf dem Fundus des zu beobachtenden Auges das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

3. Operationsmikroskop nach Anspruch 1 oder 2, **dadurch gekennzeichnet, dass** jedes Beleuchtungsteilstrahlbündel (13) derart geführt ist, dass ein zu beobachtendes Auge bezüglich jedes Beobachtungsstrahlbündels in einem Winkel von kleiner/gleich 1 Grad schräg beleuchtet ist (achsnahe Schrägbeleuchtung).

4. Operationsmikroskop nach einem der Ansprüche 1 bis 3, **dadurch gekennzeichnet, dass** die Durchmesser der Beleuchtungsspots auf dem Fundus des zu beobachtenden Auges in einem Bereich zwischen 0,5 und 1,5 mm variierbar sind.

5. Operationsmikroskop nach einem der Ansprüche 1 bis 4, **dadurch gekennzeichnet, dass** der Durchmesser der Beleuchtungsspots so ausgebildet ist, dass sie auf dem Fundus des zu beobachtenden Auges 1.5 mm, bevorzugt 1.0 mm, besonders bevorzugt 0.5 mm nicht überschreiten.

6. Operationsmikroskop nach einem der Ansprüche 1 bis 5, **dadurch gekennzeichnet, dass** die Entfernung der Mitte des Beleuchtungsspots von der Mitte der Querschnittsfläche des Beobachtungsstrahlbündels auf dem Fundus das 0.8-fache, bevorzugt das 0.5-fache, weiter bevorzugt das 0.2-fache, besonders bevorzugt das 0.05-fache des Radius der Querschnittsfläche des Beobachtungsstrahlbündels auf dem Fundus beträgt.

7. Operationsmikroskop nach einem der Ansprüche 1 bis 6, **dadurch gekennzeichnet, dass** dieses ein Objektivelement (10) aufweist.

8. Operationsmikroskop nach Anspruch 7, **dadurch gekennzeichnet, dass** das Objektivelement (10) als dessen Hauptobjektiv ausgebildet ist.

9. Operationsmikroskop nach einem der Ansprüche 1 bis 8, **dadurch gekennzeichnet, dass** Mittel vorgesehen sind, um jeweils ein Beobachtungsstrahlbündel und ein Beleuchtungsteilstrahlbündel (13) beziehungsweise das Beleuchtungsstrahlbündel (12) zu überlagern.

10. Operationsmikroskop nach Anspruch 9, soweit auf einen der Ansprüche 7 oder 8 rückbezogen, **dadurch gekennzeichnet, dass** die Mittel zum Überlagern derart angeordnet sind, dass eine Überlagerung von Beobachtungsstrahlbündel und Beleuchtungsteilstrahlbündel (13) beziehungsweise Beleuchtungsstrahlbündel (12) oberhalb des Objektivelements erfolgt.

11. Operationsmikroskop nach Anspruch 9, soweit auf einen der Ansprüche 7 oder 8 rückbezogen, **dadurch gekennzeichnet, dass** die Mittel zum Überlagern derart angeordnet sind, dass eine Überlagerung von Beobachtungsstrahlbündel und Beleuchtungsteilstrahlbündel (13) beziehungsweise Beleuchtungsstrahlbündel (12) unterhalb des Objektivelements erfolgt.

12. Operationsmikroskop nach Anspruch 11, **dadurch gekennzeichnet, dass** das Objektivelement als Varioskopoptik ausgebildet ist.

13. Operationsmikroskop nach einem der Ansprüche 9 bis 12, **dadurch gekennzeichnet, dass** die Mittel zum Überlagern wenigstens ein optisches Element in Form eines Prismas und/oder einer Strahlteilerplatte und/oder eines teildurchlässigen Spiegels und/oder eines durchbohrten Spiegels aufweisen.

14. Operationsmikroskop nach einem der Ansprüche 1 bis 13, **dadurch gekennzeichnet, dass** Mittel vorgesehen sind, um wenigstens ein ringförmiges Beleuchtungsteilstrahlbündel zu erzeugen, das um ein Beobachtungsstrahlbündel herum angeordnet ist.

15. Operationsmikroskop nach einem der Ansprüche 1 bis 14, **dadurch gekennzeichnet, dass** zwei oder mehr Lichtquellen vorgesehen sind und dass mittels jeder Lichtquelle ein Beleuchtungsteilstrahlbündel (13) erzeugt wird.

16. Operationsmikroskop nach einem der Ansprüche 1 bis 14, **dadurch gekennzeichnet, dass** eine einzige Lichtquelle vorgesehen ist und dass Mittel (11) zum Aufteilen des Beleuchtungsstrahlbündels (12) der Lichtquelle in die zwei oder mehr Beleuchtungsteilstrahlbündel (13) vorgesehen sind.

17. Operationsmikroskop nach einem der Ansprüche 1 bis 16, **dadurch gekennzeichnet, dass** die wenigstens eine Lichtquelle als Lampe, insbesondere als Halogenlampe oder Xenonlampe, als Laser, als nicht thermischer Strahler, als Lichtleiter, insbesondere als Faserlichtleiterbündel, als wenigstens eine LED oder wenigstens eine OLED ausgebildet ist.

## Claims

1. Surgical microscope for ophthalmology, having one, two or more stereoscopic observation beam paths with, in each case, an observation beam and having an illumination device, having at least one light source for producing at least one illumination beam (12) for illuminating an eye to be observed, wherein at least two illumination partial beams (13) are provided and wherein each illumination partial beam (13) extends coaxially to a stereoscopic observation beam, i.e., at exactly zero degrees or in the form of a near-axis oblique illumination at an angle of less than/equal to two degrees, wherein at least one apparatus (14) for modifying the beam cross section of at least one illumination partial beam (13) is provided, wherein the apparatus (14) is embodied as an iris stop or as a zoom system, and wherein the apparatus (14) is embodied such that the illumination partial beams (13) are embodied in such a way that they form two or more illumination spots, which are variable in size, on the fundus of an eye to be observed, wherein the illumination spots have a round geometry and wherein the diameters of the illumination spots are adjustable.

2. Surgical microscope according to Claim 1, **characterized in that** the illumination partial beams (13) are embodied in such a way that the size of the illumination spots on the fundus of the eye to be observed does not exceed 1-times, preferably 0.7-times, more preferably 0.5-times, particularly preferably 0.3-times the cross-sectional area of the observation beam on the fundus.

3. Surgical microscope according to Claim 1 or 2, **characterized in that** each illumination partial beam (13) is guided in such a way that an eye to be observed is illuminated at an angle of less than/equal to 1 degree in respect of each observation beam (near-axis oblique illumination).

4. Surgical microscope according to any one of Claims 1 to 3, **characterized in that** the diameters of the illumination spots on the fundus of the eye to be observed are variable in a range between 0.5 and 1.5 mm.

5. Surgical microscope according to any one of Claims 1 to 4, **characterized in that** the diameters of the illumination spots are embodied in such a way that they do not exceed 1.5 mm, preferably 1.0 mm, particularly preferably 0.5 mm on the fundus of the eye to be observed.

6. Surgical microscope according to any one of Claims 1 to 5, **characterized in that** the distance of the centre of the illumination spot from the centre of the cross-sectional area of the observation beam on the fundus is 0.8-times, preferably 0.5-times, more preferably 0.2-times, particularly preferably 0.05-times the radius of the cross-sectional area of the observation beam on the fundus.

7. Surgical microscope according to any one of Claims 1 to 6, **characterized in that** it has an objective element (10).

8. Surgical microscope according to Claim 7, **characterized in that** the objective element (10) is embodied as a main objective thereof.

9. Surgical microscope according to any one of Claims 1 to 8, **characterized in that** means are provided in order to superimpose an observation beam and an illumination partial beam (13) or the illumination beam (12) in each case.

10. Surgical microscope according to Claim 9, provided this refers back to either of Claims 7 and 8, **characterized in that** the means for superimposition are arranged in such a way that the superimposition of observation beam and illumination partial beam (13) or illumination beam (12) is implemented above the objective element.

11. Surgical microscope according to Claim 9, provided this refers back to either of Claims 7 and 8, **characterized in that** the means for superimposition are arranged in such a way that the superimposition of observation beam and illumination partial beam (13) or illumination beam (12) is implemented below the objective element.

12. Surgical microscope according to Claim 11, **characterized in that** the objective element is embodied as a varioscope optical unit.

13. Surgical microscope according to any one of Claims 9 to 12, **characterized in that** the means for superimposition have at least one optical element in the form of a prism and/or a beam splitter plate and/or a partly transmissive mirror and/or a pierced mirror.

14. Surgical microscope according to any one of Claims 1 to 13, **characterized in that** means are provided to produce at least one ring-shaped illumination partial beam that is arranged around an observation beam.

15. Surgical microscope according to any one of Claims 1 to 14, **characterized in that** two or more light sources are provided and **in that** an illumination partial beam (13) is produced by each light source.

16. Surgical microscope according to any one of Claims 1 to 14, **characterized in that** a single light source is provided and **in that** means (11) for splitting the illumination beam (12) of the light source into the two or more illumination partial beams (13) are provided.

17. Surgical microscope according to any one of Claims 1 to 16, **characterized in that** the at least one light source is embodied as a lamp, in particular as a halogen lamp or xenon lamp, as a laser, as a non-thermal emitter, as an optical waveguide, in particular as a fibre optic bundle, as at least one LED or at least one OLED.

## Revendications

1. Microscope chirurgical pour l'ophtalmologie, comprenant une, deux ou plusieurs trajectoires de rayons d'observation stéréoscopiques comprenant respectivement un faisceau de rayons d'observation et un dispositif d'éclairage comportant au moins une source lumineuse destinée à générer au moins un faisceau de rayons d'éclairage (12) permettant d'éclairer un oeil à observer,
dans lequel il est prévu au moins deux faisceaux de rayons d'éclairage partiels (13), et dans lequel chaque faisceau partiel de rayons d'éclairage (13) s'étend coaxialement, c'est-à-dire exactement à zéro degré ou sous la forme d'un éclairage oblique proche de l'axe sous un angle inférieur ou égal à 2 degrés par rapport à un faisceau de rayons d'observation stéréoscopique, dans lequel il est prévu au moins un dispositif (14) destiné à modifier la section transversale de faisceau d'au moins un faisceau de rayons partiel d'éclairage (13), dans lequel le dispositif (14) est réalisé sous la forme d'un diaphragme d'iris ou d'un système de zoom, et dans lequel le dispositif (14) est réalisé de manière à ce que les faisceaux de rayons partiels d'éclairage (13) soient réalisés de façon à former deux ou plusieurs points d'éclairage de taille variable sur le fond d'un oeil à observer, dans lequel les points d'éclairage présentent une géométrie ronde et les diamètres des points d'éclairage sont réglables.

2. Microscope chirurgical selon la revendication 1, **caractérisé en ce que** les faisceaux de rayons partiels d'éclairage (13) sont conçus de manière à ce que la taille des points d'éclairage sur le fond de l'oeil à observer ne dépasse pas 1 fois, de préférence 0,7 fois, de manière plus préférable 0,5 fois, de manière particulièrement préférable 0,3 fois, la surface en coupe transversale des faisceaux de rayons d'observation sur le fond de l'oeil.

3. Microscope chirurgical selon la revendication 1 ou 2, **caractérisé en ce que** chaque faisceau de rayons partiel d'éclairage (13) est guidé de manière à ce qu'un oeil à observer soit éclairé obliquement selon un angle de 1 degré ou moins par rapport à chaque faisceau de rayons d'observation (éclairage oblique près de l'axe).

4. Microscope chirurgical selon l'une des revendications 1 à 3, **caractérisé en ce que** les diamètres des points d'éclairage sur le fond de l'oeil à observer peuvent varier dans une plage comprise entre 0,5 et 1,5 mm.

5. Microscope chirurgical selon l'une des revendications 1 à 4, **caractérisé en ce que** le diamètre des points d'éclairage est tel qu'ils ne dépassent pas 1,5 mm, de préférence 1,0 mm et de manière particulièrement préférable 0,5 mm sur le fond de l'oeil à observer.

6. Microscope chirurgical selon l'une des revendications 1 à 5, **caractérisé en ce que** la distance du centre du point d'éclairage par rapport au centre de la coupe transversale du faisceau de rayons d'observation sur le fond de l'oeil est de 0,8 fois, de préférence 0,5 fois, de manière plus préférable 0,2 fois et de manière particulièrement préférable 0,05 fois le rayon de la section transversale du faisceau de rayons d'observation sur le fond de l'oeil.

7. Microscope chirurgical selon l'une des revendications 1 à 6, **caractérisé en ce qu'**il comprend un élément d'objectif (10).

8. Microscope chirurgical selon la revendication 7, **caractérisé en ce que** l'élément d'objectif (10) est réalisé sous la forme de son objectif principal.

9. Microscope chirurgical selon l'une des revendications 1 à 8, **caractérisé en ce qu'**il est prévu des moyens destinés à superposer respectivement un faisceau de rayons d'observation et un faisceau de rayons partiel d'éclairage (13) ou le faisceau de rayons d'éclairage (12).

10. Microscope chirurgical selon la revendication 9, dans la mesure où elle se réfère à l'une des revendications 7 ou 8, **caractérisé en ce que** les moyens de superposition sont disposés de manière à ce qu'une superposition du faisceau de rayons d'observation et du faisceau de rayons partiel d'éclairage (13) ou du faisceau de rayons d'éclairage (12) ait lieu au-dessus de l'élément objectif.

11. Microscope chirurgical selon la revendication 9, dans la mesure où elle se réfère à l'une des revendications 7 ou 8, **caractérisé en ce que** les moyens de superposition sont disposés de manière à ce qu'une superposition du faisceau de rayons d'observation et du faisceau de rayons partiel d'éclairage (13) ou du faisceau de rayons d'éclairage (12) ait lieu en dessous de l'élément objectif.

12. Microscope chirurgical selon la revendication 11, **caractérisé en ce que** l'élément d'objectif est réalisé sous la forme d'une optique varioscopique.

13. Microscope chirurgical selon l'une des revendications 9 à 12, **caractérisé en ce que** les moyens de superposition comprennent au moins un élément optique sous la forme d'un prisme et/ou d'une lame séparatrice de faisceau et/ou d'un miroir partiellement transparent et/ou d'un miroir percé.

14. Microscope chirurgical selon l'une des revendications 1 à 13, **caractérisé en ce qu'**il est prévu des moyens pour générer au moins un faisceau de rayons partiel d'éclairage annulaire disposé autour d'un faisceau de rayons d'observation.

15. Microscope chirurgical selon l'une des revendications 1 à 14, **caractérisé en ce qu'**il est prévu deux sources lumineuses ou plus et **en ce qu'**un faisceau de rayons partiel d'éclairage (13) est généré au moyen de chaque source lumineuse.

16. Microscope chirurgical selon l'une des revendications 1 à 14, **caractérisé en ce qu'**il est prévu une seule source lumineuse et **en ce qu'**il est prévu des moyens (11) destinés à diviser le faisceau de rayons d'éclairage (12) de la source lumineuse en lesdits deux ou plusieurs faisceaux de rayons partiels d'éclairage (13).

17. Microscope chirurgical selon l'une des revendications 1 à 16, **caractérisé en ce que** ladite au moins une source lumineuse est réalisée sous la forme d'une lampe, en particulier d'une lampe à halogène ou au xénon, d'un laser, d'un élément rayonnant non thermique, d'un guide optique, notamment d'un faisceau de fibres optiques, d'au moins une LED ou d'au moins une OLED.
